# EUROPÄISCHE PATENTSCHRIFT

(11) **EP 1 214 923 B1**
(45) Veröffentlichungstag und Bekanntmachung des Hinweises auf die Patenterteilung: **02.07.2003**
(21) Anmeldenummer: 00127398.6
(22) Anmeldetag: 14.12.2000
(51) Int. Cl.: A61H 33/02, A61H 33/14, B23K 7/10

(54) **Gas-Behandlungsgerät**
Gas-treatment apparatus
Dispositif de traitment au gas

(43) Veröffentlichungstag der Anmeldung: 19.06.2002
(73) Patentinhaber: Haaf, Frieder, 76593 Gernsbach (DE); Haaf, Karin, 76593 Gernsbach (DE)
(72) Erfinder: Haaf, Frieder, 76593 Gernsbach (DE); Haaf, Karin, 76593 Gernsbach (DE)
(74) Vertreter: Hellmayr, Wolfgang, Dr. rer. nat.

(56) Entgegenhaltungen:
- WO-A-00/01341
- DE-U- 29 820 187
- US-A- 4 323 090

## Beschreibung

Die Erfindung betrifft ein Gas-Behandlungsgerät, insbesondere ein tragbares und fahrbares Gas-Behandlungsgerät zur Behandlung des Körpers mit einem Gas, insbesondere mit Kohlendioxid (CO₂ ).

Es ist bekannt, daß im CO₂ -Bad eine Dilatation der Blutgefäße und damit eine bessere Durchblutung erfolgt. Dies ist besonders auf die Erweiterung der den Kapillaren vorgeschalteten Arteriolen zurückzuführen. Das perkutan resorbierte CO₂-Gas setzt den Tonus der glatten Muskulatur der terminalen und präterminalen Arteriolen herab.

Die perkutane Wirkung von CO₂, die einerseits entspannend und andererseits stimulierend ist, kann auf vielfältige Art im kosmetischen und medizinischen Bereich und für Gymnastikzwecke, in Fitness-Studios und Massagepraxen nutzbar gemacht werden.

Es wurden schon CO₂-Behandlungen und eine Permeation im CO₂-Wasserbad durchgeführt. Dabei wird aber eine Einwirkung durch das Gas in nur relativ niedriger Konzentration erreicht. Bekannt sind offene und geschlossene Wannenbäder. Bei den ersteren besteht die Gefahr, daß aus dem Bad entweichende Gase in zu großer Menge eingeatmet werden, was zu Kopfschmerzen oder in extremen Fällen zum Ersticken führen kann. Daher hat man geschlossenen Wannenbäder vorgeschlagen, bei denen das Gesicht der zu behandelnden Person vor dem aus dem Bad entweichenden CO₂ dadurch geschützt wird, daß die Wanne abgedeckt ist und die Abdeckschürze den Hals dicht umschließt.

In höherer Konzentration kann das CO₂ zur Einwirkung gebracht werden, wenn es in Gasform zugeführt wird. Hierzu wird zweckmäßigerweise der zu behandelnde Körperteil mit einer gasdichten, mit CO₂-Gas gefüllten Hülle umschlossen. Eine toxikologisch relevante Konzentration des Gases in der Atemluft wird hierdurch ausgeschlossen. Diese Methode der gasförmigen Anwendung ist unter verschiedenen Gesichtspunkten vorteilhafter als die Anwendung des CO₂ in wäßriger Lösung. So erfolgt die Resorption des CO₂ schneller und wirksamer, die Behandlung kann bei Umgebungstemperatur vorgenommen werden, und das Gasbad mit Hilfe der Hülle ist wirtschaftlicher als das Wannenbad.

Das Problem, das diesen bekannten Vorrichtungen zur CO₂ -Behandlung zugrunde liegt, ist aber, daß die Behandlung nur in dem speziell eingerichteten Behandlungsraum, nicht aber, jedenfalls nicht ohne besonderen Aufwand, direkt auf der Station am Bett oder vom Patienten selbst in seiner Wohnung oder an einem beliebigen gewünschten Ort vorgenommen werden kann. Das Wannenbad ist in der Regel in einem Badezimmer lokalisiert und kann nicht ohne größeren Aufwand an einen anderen Ort gebracht werden. Bei der gasgefüllten Hülle wird nicht nur eine CO₂-Quelle, z. B. eine Gasflasche, sondern in vielen Fällen, z. B. bei der Wundheilung, ein spezielles Lagerungskissen benötigt, um das zu behandelnde Körperglied abzustützen.

Ein verbesserter tragbarer Gas-Behandlungssatz ist aus der deutschen Gebrauchsmusterschrift 298 20 187.9 bekannt. Dieser Gas-Behandlungssatz umfaßt einen Koffer, ein Gasvorratsgefäß, mindestens eine schlauch- und/ oder beutelartige (bezw. tütenartige) Hülle, die hinsichtlich ihrer Form einem Körperteil anatomisch angepaßt ist, mindestens eine Dichtung zum gasdichten Anlegen der Hülle, mindestens eine Gasleitung zum Füllen der Hülle mit dem Gas und mindestens ein Lagerungskissen.

Dieser Gas-Behandlungssatz ist gegenüber älteren Gas-Behandlungsvorrichtungen vor allem insofern vorteilhaft, als er zusammen mit dem/den Lagerungskissen leicht zu transportieren ist. Dadurch und, weil sämtliche benötigten Instrumente und Mittel Teile des Behandlungssatzes sind, wird ermöglicht, daß der Patient nicht nur im speziellen Behandlungszimmer, sondern auch am Bett in der Station oder zu Hause, wo normalerweise keine Gasquelle als installierte Leitung oder Kompressionsflasche vorhanden ist, behandelt werden kann.

Zudem kann die Behandlung auch von dem Patienten selbst ohne Kontrolle durch Fachkräfte gefahrlos durchgeführt werden. Der Patient kann während der Behandlung sitzen, stehen oder liegen. Die aus Folien gewonnenen Hüllen können nach der Behandlung leicht und umweltfreundlich entsorgt werden.

Nachteile des aus der deutschen Gebrauchsmusterschrift 298 20 187.9 bekannten, tragbaren Gas-Behandlungssatzes sind aber, daß die Bedienung der Gasflasche nicht von außerhalb des Gehäuses ohne vorhergehendes Öffnen des Gehäuses möglich ist und daß das Füllen der Hülle bezw. des Beutels (der Tüte) über dem zu behandelnden Körperteil mit dem Behandlungsgas in manchen Fällen nicht so glatt und schnell vonstatten geht, wie es wünschenswert wäre.

Die Aufgabe, die sich dem Erfinder stellte, war also die, eine Vorrichtung bereitzustellen, die es ermöglicht, Personen zum Zwecke der Entspannung, Lockerung, Stimulierung, Heilung oder ähnlichen Zwecken mit einem geeigneten Gas, z. B. CO₂ zu behandeln, welche Vorrichtung besonders einfach zu handhaben und ohne besonderen Aufwand transportabel, also tragbar und/oder fahrbar sein sollte, und mit der auch das Füllen der Hülle, z. B. eines Beutels (bezw. einer Tüte), mit dem Behandlungsgas einfacher und sicherer möglich sein sollte.

Dieses Problem wird mit den in den Patentansprüchen aufgeführten Merkmalen gelöst.

Gegenstand der Erfindung ist nun ein Gas-Behandlungsgerät, umfassend ein Gehäuse (1) mit mindestens einem aufstellbaren Deckel (2), mindestens einer Vordertür (3) und Rädern und/oder Rollen (4), mindestens eine Gasflasche (5), eine entsprechende Flaschenbefestigung (6), mindestens einem Druckregler (7), Staufächern (8), welches Gas-Behandlungsgerät dadurch gekennzeichnet ist, daß in den Deckel (2) (Paneel) eine von außen bedienbare Gasflaschenventil-Bedienungsvorrichtung (9) und Anzeigeinstrumente (10) eingelassen sind, wobei die Gasflaschenventil-Bedienungsvorrichtung (9) bei geschlossenem Deckel (2) mit ihrem unteren Ende in das Gasflaschenventilrad (11) eingreift, und daß in dem Gehäuse (1) ein herausnehmbares Gasauslaßhandstück (12) untergebracht ist, das über eine Schlauchleitung (13) mit dem Gasflaschenventilrad (11) verbunden ist.

In einer bevorzugten Ausführungsform ist die Gasflaschenventil-Bedienungsvorrichtung (9) als Drehknauf ausgebildet, der auf einer Welle sitzt, die auf der Innenseite des Deckels (2) am Ende eine Kupplung (14) aufweist, welche bei geschlossenem Deckel (2) in das Ventilrad (11) eingreift.

Die Bedienung der Gasflasche, die vorzugsweise eine Kohlendioxidflasche ist, kann in einfacher Weise von außen her über beispielsweise Bolzen (15), vorzugsweise zwei Bolzen, welche auf einer Scheibe am unteren Ende der Welle sitzen und als Kupplung (14) dienen, indem sie bei geschlossenem Deckel (Paneel) in das Gasflaschenventilrad (11) eingreifen und die Drehbewegung des Drehknaufs der Gasflaschenventil-Bedienungsvorrichtung (9) auf das Gasflaschenventilrad (11) übertragen.

Das Gasauslaßhandstück (Pistole) (12) kann beispielsweise in der Tür innenseitig untergebracht sein. Es kann aber auch in einer der Seitenwände untergebracht sein, wobei es zweckmäßig ist, daß man in dieser Seitenwand eine von außen zu öffnende Klappe vorsieht, die so ausgestaltet und angebracht ist, daß ein bequemes Herausnehmen der Pistole möglich ist.

Das Gehäuse (1) ist im allgemeinen, um es besser transportieren zu können, mit einem oder mehr als einem Griff ausgestattet, der vorzugsweise als Ausziehgriff ausgebildet ist (20).

Als Gasflasche (Gasvorratsgefäß, Druckflasche) (5) kann eine handelsübliche Gasflasche (mit einem Inhalt von z. B. 3,5 oder 2 kg) verwendet werden. Gegebenenfalls ist an geeigneter Stelle des Gasstroms ein Bakterienfilter vorgesehen.

Gegenstand der Erfindung ist auch die Verwendung des erfindungsgemäßen Gas-Behandlungsgerätes zum Befüllen von Hüllen, welche Verwendung dadurch gekennzeichnet ist, daß man die Hülle durch ein in die Hülle eingesetztes Ventil (16) befüllt, welches Ventil (16) vorzugsweise aus einem Unterteil (17), das von innen nach außen durch die Hülle gesteckt ist, einem Schlauchstück (18), das von außen auf das Unterteil(17) aufgezogen ist, und einer Verschlußklemme (19) zum Verschließen des Ventils (16) besteht.

Die Verwendung eines solchen Ventils (16) erleichtert nicht nur das Befüllen der Hülle mit dem Behandlungsgas, das vorzugsweise CO₂ ist, sondern auch die Befestigung der Hülle, bezw. des Beutels (der Tüte) oder des Schlauchs am Körper, beispielsweise mit Hilfe von Wundpflastern, mit welchen Plastikhüllen bezw. -beutel an den Rändern auf der Haut gasdicht aufgeklebt werden.

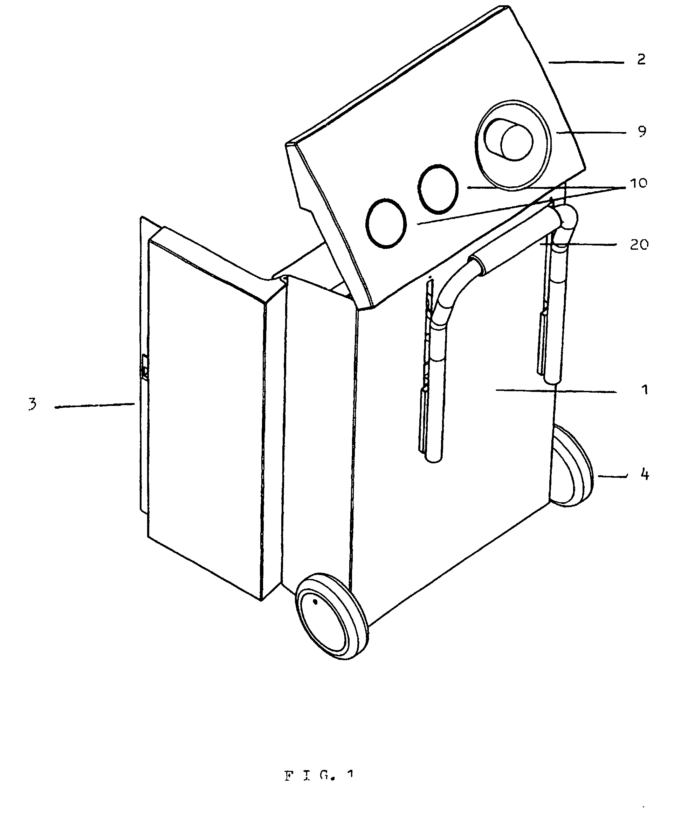

Ein Ausführungsbeispiel der Erfindung wird anhand der Figuren 1 bis 7 erläutert. Es zeigen:

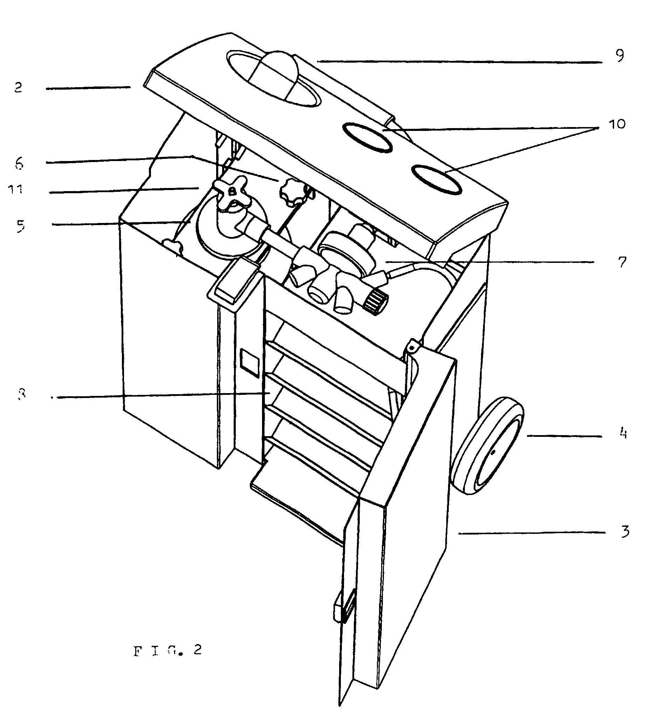

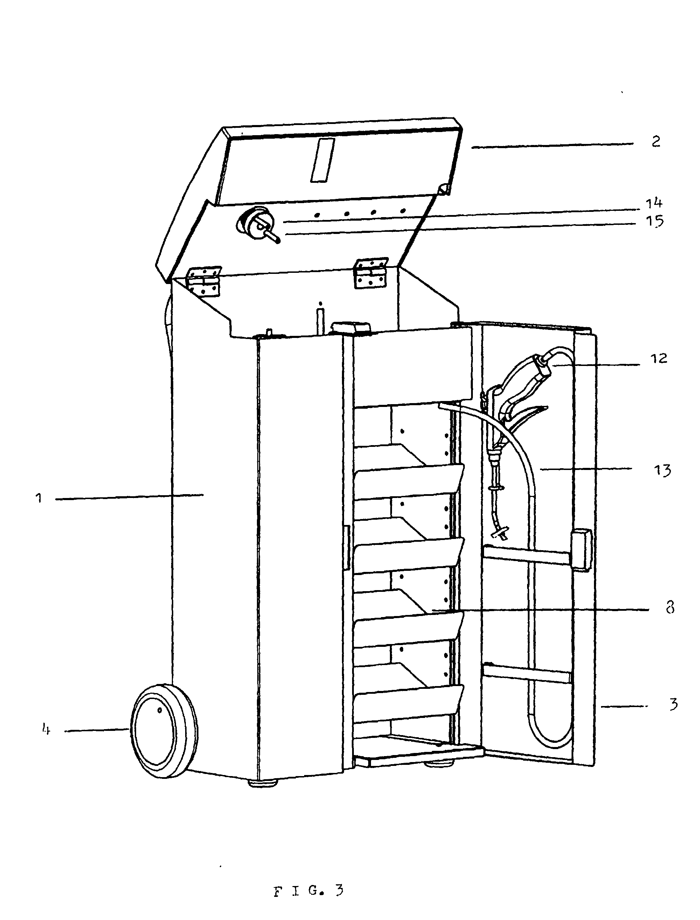

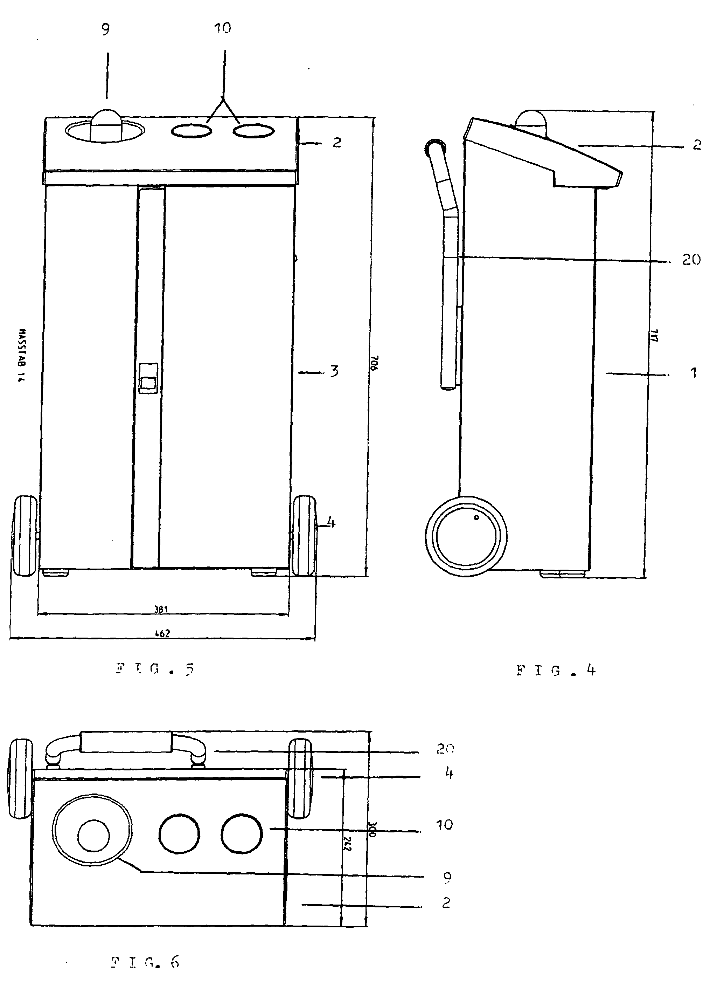

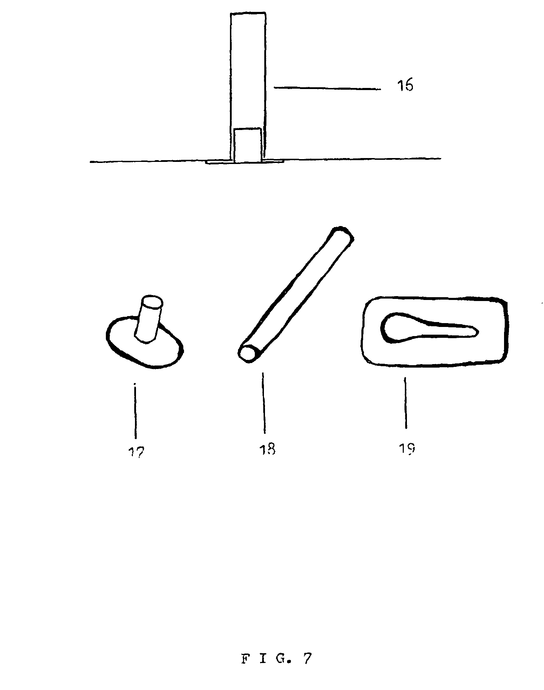

Fig. 1 das Gas-Behandlungsgerät geöffnet von hinten, Fig. 2 dasselbe Gerät geöffnet von vorne; Fig. 3 dasselbe Gerät geöffnet von vorne, aber aus einer anderen Perspektive; Fig. 4 dasselbe Gerät geschlossen in einer Seitenansicht von rechts, Fig. 5 dasselbe Gerät geschlossen von vorne, Fig. 6 dasselbe Gerät geschlossen von oben, und Fig. 7 das Ventil für die Hülle.

Die Ziffern in den Figuren sowie in der Beschreibung, in den Patentansprüchen und in der Zusammenfassung haben die nachstehenden Bedeutungen:

### Legende:

- 1: Gehäuse
- 2: Deckel
- 3: Vordertür
- 4: Räder (bezw. Rollen)
- 5: Gasflasche
- 6: Flaschenbefestigung
- 7: Druckregler
- 8: Staufächer
- 9: Gasflaschenventil-Bedienungsvorrichtung
- 10: Anzeigeinstrument
- 11: Gasflaschenventilrad
- 12: Gasauslaß-Handstück (Pistole)
- 13: Schlauchleitung
- 14: Kupplung
- 15: Bolzen
- 16: Hüllenventil
- 17: Unterteil des Ventils (16)
- 18: Schlauchstück für das Ventil (16)
- 19: Verschlußklemme
- 20: Ausziehgriff

Das Gehäuse (1) besteht zweckmäßigerweise aus Aluminium oder Edelstahl, kann aber aus beliebigen anderen geeigneten Werkstoffen hergestellt sein, solange die Anforderungen an die Hygiene und Keimfreiheit erfüllt sind.

Das erfindungsgemäße Gas-Behandlungsgerät ist gegenüber den zum Stand der Technik gehörenden Gas-Behandlungsvorrichtungen vor allem insofern vorteilhaft, als es sehr bequem und einfach zu bedienen und leicht zu transportieren ist und.sehr verschiedenen Anwendungszwecken zugeführt werden kann. Alle benötigten Instrumente und Hilfsgegenstände, wie Pflaster, gasdichte Hüllen, Beutel oder Tüten, mit den dazugehörenden Hüllenventilen (16) und Verschlußklemmen (19), Ersatzschläuche, Klebebänder u.s.w. können in den Staufächern (8) untergebracht werden und sind leicht zugänglich. Dadurch wird es ermöglicht, den Patienten nicht nur im speziellen Behandlungszimmer, sondern auch am Bett in der Station oder zu Hause, wo normalerweise keine Gasquelle als installierte Leitung und keine Kompressionsflasche vorhanden ist, zu behandeln.

Zudem kann die Behandlung auch von dem Patienten selbst ohne Kontrolle durch Fachkräfte gefahrlos durchgeführt werden. Der Patient kann während der Behandlung sitzen, stehen oder liegen.

Eine Einweg-Variante umfaßt ein Set aus Beuteln und einer kleinen Gaskartusche, auf der ein Kopf aufgeschraubt ist, der einen Druckminderer sowie eine Pistole für den Gasauslaß enthält. Mit diesem Minisystem aus Gaskartusche, Druckminderer und Gasauslaßhandstück (Pistole) können die Beutel befüllt werden, und die Kartuschen können vom Patienten oder den Pflegekräften der Sozialstationen und/oder ambulantem Pflegepersonal gewechselt werden.

Mit dem neuen Gas-Behandlungsgerät können bestimmte Körperteile gezielt behandelt werden. Die Behandlungszeit beträgt im Falle der bevorzugten CO₂-Behandlung beispielsweise 15 bis 30 Minuten. Diese Behandlungsdauer hat sich als zweckmäßig erwiesen, kann aber im allgemeinen komplikationslos unter- oder überschritten werden. Die Behandlungstempertur kann die Umgebungstemperatur sein; erhöhte oder erniedrigte Temperaturen im Bereich von 10 bis 40 Grad C. sind ebenfalls anwendbar.

Das erfindungsgemäße Gas-Behandlungsgerät kann auf verschiedenen Gebieten verwendet werden. Dazu gehören die medizinische Behandlung ( z. B. von Erysipel, Ulcus cruris, Durchblutungsstörungen), sportmedizinische Therapie und kosmetische Behandlung. Die Wundheilung läßt sich beschleunigen.

International festgelegte Indikationen für die CO₂ -Therapie sind in der folgenden Liste enthalten:
Arterielle Verschlußkrankheiten in jedem Stadium;
Mikroangiopathie jeder Genese;
Polyneuropathie, speziell malum perforans;
zerebrale Durchblutungsstörungen;
arterielle Hypertonie;
Algodystrophie (morbus Sudeck);
vegetative Dystonie;
Venöse Insuffizienz, venöse Ulcera;
Raynaud-Syndrom;
Sklerodermie; und
Dekubital-Ulcera.

## Patentansprüche

1. Gas-Behandlungsgerät, umfassend ein Gehäuse (1) mit mindestens einem aufstellbarem Deckel (2), mindestens einer Vordertür (3) und Rädern und/oder Rollen (4), mindestens eine Gasflasche (5), eine entsprechende Flaschenbefestigung (6), mindestens einem Druckregler (7), Staufächern (8), **dadurch gekennzeichnet, daß** der Deckel (2) als Paneele ausgebildet ist, in welche eine von außen bedienbare Gasflaschenventil-Bedienungsvorrichtung (9) und Anzeigeinstrumente (10) eingelassen sind, wobei die Gasflaschenventil-Bedienungsvorrichtung (9) bei geschlossenem Deckel (2) mit ihrem unteren Ende in das Gasflaschenventilrad (11) eingreift, und daß in dem Gehäuse (1) ein herausnehmbares Gasauslaßhandstück (12) untergebracht ist, das über eine Schlauchleitung (13) mit dem Gasflaschenventilrad (11) verbunden ist.

2. Gas-Behandlungsgerät nach Anspruch 1, **dadurch gekennzeichnet, daß** die Gasflaschenventil-Bedienungsvorrichtung (9) als Drehknauf ausgebildet ist, der auf einer Welle sitzt, die auf der Innenseite des Deckels (2) am Ende eine Kupplung (14) aufweist, welche bei geschlossenem Deckel (2) in das Ventilrad (11) eingreift.

3. Gas-Behandlungsgerät nach Anspruch 2, **dadurch gekennzeichnet ist, daß** die in das Ventilrad (11) eingreifende Kupplung (14) Bolzen (15) aufweist.

4. Gas-Behandlungsgerät nach Anspruch 3, **dadurch gekennzeichnet ist, daß** zwei Bolzen vorhanden sind.

5. Gas-Behandlungsgerät nach einem der Ansprüche 1 bis 4, **dadurch gekennzeichnet, daß** das Gasauslaßhandstück (12) in der Tür innenseitig untergebracht ist

6. Gas-Behandlungsgerät nach einem der Ansprüche 1 bis 4, **dadurch gekennzeichnet, daß** das Gasauslaßhandstück (12) in einer der Seitenwände des Gehäuses (1), untergebracht ist und durch eine in der Seitenwand angebrachte Klappe herausgenommen werden kann.

7. Gas-Behandlungsgerät nach einem der Ansprüche 1 bis 6, **dadurch gekennzeichnet, daß** die Gasflasche (5) eine Kohlendioxidflasche ist.

8. Verwendung des Gas-Behandlungsgerätes nach einem der Ansprüche 1 bis 7 zum Befüllen einer gasdichten Hülle mit einem Gas, **dadurch gekennzeichnet, daß** man die Hülle durch ein in sie eingesetztes Ventil befüllt.

9. Verwendung des Gas-Behandlungsgerätes nach Anspruch 8, **dadurch gekennzeichnet, daß** die gasdichte Hülle ein Beutel und/oder Schlauch ist.

10. Verwendung des Gas-Behandlungsgerätes nach Anspruch 8 oder 9, **dadurch gekennzeichnet, daß** das in die Hülle eingesetzte Ventil aus einem Unterteil (17), das von innen nach außen durch die Hülle gesteckt ist, einem Schlauchstück (18), das von außen auf das Unterteil (17) aufgezogen ist, und einer Verschlußklemme (19) zum Verschließen des Ventils (16) besteht.

## Claims

1. An apparatus for gas treatment comprising a housing (1) having at least one hingeable lid (2), at least one door (3) at its front, wheels and/or rollers (4), at least one gas cylinder (5), a corresponding cylinder mounting (6), at least one pressure regulator (7), and storage compartments (8),
**characterized in that**
lid (2) is configured as a panel into which an externally-operable gas cylinder valve control device (9) and display indicators (10) are recessed, wherein the lower end of said gas cylinder valve control device (9) engages with gas cylinder valve wheel (11) when lid (2) is closed, and that a removable gas discharger hand piece (12) is accommodated within said housing (1) and connected to gas cylinder valve wheel (11) by means of a hose (13).

2. The apparatus for gas treatment in accordance with claim 1,
**characterized in that**
gas cylinder valve control device (9) is configured as a twistable knob seated on a shaft having a coupling (14) at its terminus inside lid (2) which engages with valve wheel (11) when lid (2) is closed.

3. The apparatus for gas treatment in accordance with claim 2,
**characterized in that**
coupling (14) engaging with valve wheel (11) comprises bolts (15).

4. The apparatus for gas treatment in accordance with claim 3,
**characterized in that**
two bolts are provided.

5. The pparatus for gas treatment in accordance with one of claims 1-4,
**characterized in that**
said gas discharger hand piece (12) is accommodated at the inside of the door.

6. The apparatus for gas treatment according to one of claims 1-4,
**characterized in that**
gas discharger hand piece (12) is accommodated in one of the side panels of said housing (1) and can be removed by means of a flap affixed to said side panel.

7. The apparatus for gas treatment according to one of claims 1-6,
**characterized in that**
gas cylinder (5) is a carbon dioxide cylinder.

8. A use of an apparatus for gas treatment in accordance with one of claims 1-7 for filling a gas into a gas-tight casing,
**characterized in that**
said casing is filled via a valve inserted therein.

9. The use of an apparatus for gas treatment in accordance with claim 8,
**characterized in that**
said gas-tight casing is a pouch and/or tube.

10. The use of an apparatus for gas treatment in accordance with claim 8 or 9,
**characterized in that**
the valve inserted in said casing consists of a base member (17) pushed outwardly through said casing from the interior, a unit of tubing (18) pulled over said base member (17) from the outside, and a sealing clamp (19) for shutting said valve (16).

## Revendications

1. Appareil thérapeutique à gaz comprenant un boîtier (1) avec au moins un couvercle ajustable (2), au moins une porte avant (3) et des roues et/ou des galets (4), au moins une bouteille de gaz (5), une fixation (6) de bouteille correspondante, au moins un régulateur de pression (7), des coffrets de rangement (8),
**caractérisé en ce que**
le couvercle (2) se présente sous la forme de panneaux, dans lesquels sont encastrés un système de commande de valve de la bouteille de gaz depuis l'extérieur (9) et des indicateurs (10), le système de commande de valve de la bouteille de gaz (9) étant emboîté par son extrémité inférieure dans l'engrenage de la valve de bouteille de gaz (11) lorsque le couvercle (2) est fermé, et qu'une pièce à main amovible d'échappement de gaz (12) est placée dans le boîtier (1), laquelle est raccordée par une conduite en tuyau (13) avec l'engrenage de la valve de bouteille de gaz (11).

2. Appareil thérapeutique à gaz selon la revendication 1,
**caractérisé en ce que**
le système de commande de valve de bouteille de gaz (9) se présente sous la forme d'un robinet, fixé sur une tige, laquelle possède un coupleur (14) à l'extrémité de la face intérieure du couvercle (2), lequel s'emboîte dans l'engrenage de valve (11) lorsque le couvercle (2) est fermé.

3. Appareil thérapeutique à gaz selon la revendication 2,
**caractérisé en ce que**
le coupleur (14) qui s'emboîte dans l'engrenage de valve (11) possède des goujons (15).

4. Appareil thérapeutique à gaz selon la revendication 3,
**caractérisé en ce que**
deux goujons sont disponibles.

5. Appareil thérapeutique à gaz selon l'une des revendications 1 à 4,
**caractérisé en ce que**
la pièce à main amovible d'évacuation du gaz (12) est fixée sur la face intérieure de la porte.

6. Appareil thérapeutique à gaz selon l'une des revendications 1 à 4,
**caractérisé en ce que**
la pièce à main amovible d'évacuation du gaz (12) est disposée sur l'un des panneaux latéraux du boîtier (1) et peut être retirée à partir d'une trappe située sur le panneau latéral.

7. Appareil thérapeutique à gaz selon l'une des revendications 1 à 6,
**caractérisé en ce que**
la bouteille de gaz (5) est une bouteille de gaz carbonique.

8. Utilisation de l'appareil thérapeutique à gaz selon l'une des revendications 1 à 7 pour le remplissage d'une enveloppe étanche avec un gaz,
**caractérisée en ce que**
l'enveloppe est remplie par l'intermédiaire d'une valve qui y est installée.

9. Utilisation de l'appareil thérapeutique à gaz selon la revendication 8,
**caractérisée en ce que**
l'enveloppe étanche est un sachet et/ou un tuyau.

10. Utilisation de l'appareil thérapeutique à gaz selon l'une des revendications 8 ou 9,
**caractérisée en ce que**
la valve installée dans l'enveloppe est composée d'une pièce inférieure (17), qui est insérée de l'intérieur vers l'extérieur à travers l'enveloppe, d'un tuyau (18), qui remonte à partir de l'extérieur sur la pièce inférieure (17), et d'une borne d'obturation (19) pour la fermeture de la valve (16).
